# EUROPEAN PATENT SPECIFICATION

(11) **EP 3 096 864 B1**
(45) Date of publication and mention of the grant of the patent: **27.03.2019**
(21) Application number: 14752535.6
(22) Date of filing: 05.08.2014
(51) Int. Cl.: B01D 53/08, C07C 7/12, C10G 5/02

(54) **PROCESS FOR RECOVERING NATURAL GAS LIQUIDS FROM NATURAL GAS PRODUCED IN REMOTE LOCATIONS**
VERFAHREN ZUR RÜCKGEWINNUNG VON FLÜSSIGERDGAS AUS AN ENTFERNTEN STANDORTEN HERGESTELLTEM ERDGAS
PROCÉDÉ POUR RÉCUPÉRER DES LIQUIDES DE GAZ NATUREL À PARTIR DE GAZ NATUREL PRODUIT DANS DES ENDROITS DISTANTS

(30) Priority: 21.01.2014 US 201461929687 P
(43) Date of publication of application: 30.11.2016
(73) Proprietor: Dow Global Technologies LLC, Midland, MI 48674 (US)
(72) Inventor: MATTEUCCI, Scott T., Midland, MI 48640 (US); BADHWAR, Ajay N., Houston, TX 77007 (US); SHURGOTT, Nicholas J., West Mifflin, PA 15122 (US); GOLTZ, H. Robert, Midland, MI 48642 (US)
(74) Representative: Murgitroyd & Company
(86) International application number: PCT/US2014/049781
(87) International publication number: WO 2015/112199

(56) References cited:
- WO-A1-2009/105251
- GB-A- 638 745
- US-A- 2 527 964
- US-A- 4 040 990
- US-A- 5 079 274
- US-A1- 2011 315 012
- US-B1- 6 562 113
- "11.1" In: Frank Jahn, Mark Cook, Mark Graham: "HYDROCARBON EXPLORATION AND PRODUCTION", 1 May 2008 (2008-05-01), Elsevier, XP002731117, ISBN: 978-0-444-53236-7 vol. 55, pages 266-291, 11.1.3; page 280

## Description

### FIELD OF THE INVENTION

This invention relates to a process for separating natural gas liquids from a natural gas feedstream prior to flaring, specifically to a natural gas feedstream produced in remote locations where there are no natural gas pipelines.

### BACKGROUND OF THE INVENTION

Natural gas consists primarily of saturated hydrocarbon components such as methane, ethane, propane, butane, and heavier hydrocarbons. Natural gas typically contains about 60-100 mole percent methane, the balance being primarily heavier alkanes. Alkanes of increasing carbon number are normally present in decreasing amounts. Carbon dioxide, hydrogen sulfide, nitrogen, and other gases may also be present.

There are many reasons to separate the higher alkanes known as natural gas liquids (NGL) from natural gas to provide a methane-rich natural gas stream. One such reason is to meet pipeline specifications or liquefied natural gas (LNG) specification for heating value, dew point, and condensation. Some stationary internal combustion engines, such as natural gas engines, are designed to operate for optional efficiency within a specific BTU range and may require higher maintenance costs, higher operating temperatures, reduced equipment life expectancy, and/or generate increased pollution if operated at higher BTUs.

Additionally, it may be financially desirable to recover natural gas liquids from natural gas. NGLs including ethane, propane, butane, and lesser amounts of other heavy hydrocarbons may be used as petrochemical feedstocks where they have a higher value as compared to their value as a fuel gas component.

In other instances, gas is co-produced with oil and the concentrations of NGLs can be very high ranging from a fraction of a percent of the gas flow to tens of percent. This gas can be of poor quality due to high levels of carbon dioxide, nitrogen, and other components. The gas flow rate can be small and often it is not economical to bring a pipeline to an isolate location where natural gas is produced, such gas is sometimes referred to as stranded gas. In these instances, the best alternative is to flare the gas. However, flaring of gas high in NGLs may have a significant negative impact on the environment, accounting for a significant amount of CO₂ and heat that is injected into the atmosphere. In addition to capturing value for separated NGLs that can be stored in a tank for later transportation and sale, it would be environmentally advantageous to remove the NGLs from the gas to reduce the amount of CO₂ and heat uselessly released into the environment.

There are two basic steps for the separation of natural gas liquids from a natural gas stream. First, the liquids must be extracted from the natural gas. Second, these natural gas liquids must be separated themselves, down to their base components. The two principle techniques for removing NGLs from the natural gas stream are the oil absorption method and the cryogenic expander process. These two processes account for around 90 percent of total natural gas liquids production.

The absorption method of NGL extraction utilizes an absorbing oil which has an affinity for NGLs. Before the oil has picked up any NGLs, it is termed "lean" absorption oil. As the natural gas is passed through an absorption tower, it is brought into contact with the absorption oil which soaks up a high proportion of the NGLs. The "rich" absorption oil, now containing NGLs, exits the absorption tower through the bottom. It is now a mixture of absorption oil, propane, butanes, pentanes, and other heavier hydrocarbons. The rich oil is fed into lean oil stills, where the mixture is heated to a temperature above the boiling point of the NGLs, but below that of the oil. This process allows for the recovery of around 75 percent of butanes, and 85 to 90 percent of pentanes and heavier molecules from the natural gas stream.

Although there are many known adsorption processes, there is always a compromise between high recovery and process simplicity (i.e., low capital investment). Common adsorption technologies focus on removal of hydrocarbons, which works well in non-hydrocarbon rich streams, but is limited in applicability in hydrocarbon continuous streams. Further this technology is not selective for certain molecular size/weight.

Cryogenic processes are also used to extract NGLs from natural gas. While absorption methods can extract almost all of the heavier NGLs, the lighter hydrocarbons, such as ethane, are often more difficult to recover from the natural gas stream. In certain instances, it is economic to simply leave the lighter NGLs in the natural gas stream. However, if it is economic to extract ethane and other lighter hydrocarbons, cryogenic processes are required for high recovery rates. Essentially, cryogenic processes consist of dropping the temperature of the gas stream to around -120 degrees Fahrenheit. There are a number of different ways of chilling the gas to these temperatures, but one of the most effective is known as the turbo expander process. In this process, external refrigerants are used to cool the natural gas stream. Then, an expansion turbine is used to rapidly expand the chilled gases, which causes the temperature to drop significantly. This expansion can take place across a valve as well. This rapid temperature drop caused by the Joule-Thompson effect condenses ethane and other hydrocarbons in the gas stream, while maintaining methane in gaseous form. This process allows for the recovery of about 90 to 95 percent of the ethane originally in the natural gas stream. In addition, the expansion turbine is able to convert some of the energy released when the natural gas stream is expanded into recompressing the gaseous methane effluent, thus saving energy costs associated with extracting ethane. These plants can be called JT plants, refrig plants, or cryo plants which are all variations on the same temperature drop processes.

While reliable, cryogenic systems suffer from a number of shortcomings including high horsepower requirements. Further, such systems require relatively rigorous and expensive maintenance to function properly. Mechanical refrigeration systems also have practical limits with respect to the amount of cold that may be delivered, accordingly, the efficiency and capacity of such systems is limited. The operating window (range of operating conditions the plants can function well within) is a relatively narrow window, requires time to start-up and shut-down effectively, and is quite capitally intensive. As a result these facilities are often used at higher gas flow rates to ensure a more economic cost to treat the system. And if the facility is to be constructed, and can only operate in a narrow range of operating conditions, there are significant upstream treatment systems required to remove CO₂ (amine systems), water (glycol dehydration) and sometimes even pre-chilling (propane chillers).

Once NGLs have been removed from the natural gas stream, the mixed stream of different NGLs must be separated out. The process used to accomplish this task is called fractionation. Fractionation works based on the different boiling points of the different hydrocarbons in the NGL stream. Essentially, fractionation occurs in stages consisting of the boiling off of hydrocarbons one by one. By proceeding from the lightest hydrocarbons to the heaviest, it is possible to separate the different NGLs reasonably easily.

Of the various alternative technologies, adsorption process appears to be the most promising, for instance see WO2009105251. An adsorbent suitable for the separation of NGLs should have high adsorption capacity and selectivity for either olefin or paraffin. Adsorbed component should be able to desorb easily by simple chemical engineering operation such as by increasing the temperature or by reducing the pressure. Conventional adsorbents such as zeolites, activated carbon, activated alumina, silica gels, polymer supported silver chloride, copper-containing resins, and the like known in the prior art which exhibit selectivity for ethylene or propylene suffer from one or more drawbacks such as slow adsorption kinetics, poor adsorption capacity, and/or selectivity. Furthermore, due to ever changing business requirements and demands, it is desirable to have adsorbents exhibiting even higher adsorption capacity, selectivity, and/or reversibility for efficient separation of hydrocarbon gases.

Oil and shale fields, such as the Bakken shale fields of North Dakota, are often located in remote locations where natural gas pipelines do not exist. Remote locations combined with historically low natural gas prices and the extensive time and cost to develop pipeline networks has led to the practice known as flaring. While flaring is less harmful than releasing the raw natural gas directly to the environment, it would be desirable if the environmental impact of flaring could be reduced. Harmful environmental emissions may be reduced by lowering the amount of gas burned and/or reducing the BTU content of the gas. It would be desirable to have a method to reduce the environmental impact of flaring natural gas.

### SUMMARY OF THE INVENTION

The present invention is such a method to reduce the environmental impact of flaring natural gas and to derive value by removing and recovering some or all natural gas liquids from the natural gas prior to flaring.

One embodiment of the present invention is a method for separating and recovering some or all natural gas liquids (NGLs): ethane, propane, butane, pentane, or heavier hydrocarbons from a natural gas feedstream, preferably a natural gas feedstream from an oil well, a gas well, or a condensate well, forming a methane-rich natural gas supply and then flaring said methane-rich natural gas supply, wherein the NGLs are separated from the natural gas feedstream by means of a NGLs separation unit, wherein the NGLs separation unit comprises: (i) an adsorption unit comprising an adsorption bed comprising an adsorbent media which adsorbs NGLs to form a loaded adsorbent media and (ii) a regeneration unit comprising a means to regenerate loaded adsorbent media by causing the release of adsorbed NGLs from the loaded adsorbing media and forming regenerated adsorbent media wherein the method comprises the steps of: (a) passing the natural gas feedstream through the adsorption unit generating the adsorbent loaded with NGLs and a methane-rich natural gas supply, (b) transporting the adsorbent loaded with NGLs from the adsorption unit to the regeneration unit, (c) regenerating the adsorbent loaded with NGLs by releasing the adsorbed NGLs from the loaded adsorbing media and forming regenerated adsorbent media (d) transporting the regenerated adsorbent media back to the adsorption unit for reuse, (e) recovering the released NGLs, and (f) flaring the methane-rich natural gas supply.

In one embodiment of the method of the present invention described herein above, the recovered NGLs are stored and/or transported by truck or rail individually or as a mixture of gases (e.g., as C₂, C₃, C₄, C₅, etc.) or liquefied individually or as a mixture of liquids.

In the method of the present invention described herein above, the adsorption media is porous cross-linked polymeric adsorbents, partially pyrolized macroporous polymers, or mixtures thereof.

In the method of the present invention described herein above the loaded adsorption media is regenerated by means of reduced pressure over the media, heating the media, or a combination of reduced pressure and heating and preferably regenerated by a microwave heating system, preferably the regeneration step is operated as a batch process, a semi-continuous process, or as a continuous process.

### BRIEF DESCRIPTION OF THE DRAWINGS

**FIG. 1** is a schematic of a method of the present invention to separate NGLs from a natural gas feedstream prior to flaring.
**FIG. 2** is a schematic of a NGLs adsorption and regeneration process useful for the method of the present invention.
**FIG. 3** is a schematic of another method of the present invention to separate NGLs from a natural gas feedstream prior to flaring.
**FIG. 4** is a schematic of a NGLs adsorption and regeneration process comprising a microwave regeneration unit useful for the method of the present invention.
**FIG. 5** shows the initial and repeat sorption isotherms for butane for Example 1.
**FIG. 6** shows the initial and repeat sorption isotherms for butane for Example 2.
**FIG. 7** shows the initial and repeat sorption isotherms for propane for Example 3.
**FIG. 8** shows the sorption isotherms for methane, ethane, propane, butane, and pentane for Example 1.
**FIG. 9** shows the sorption isotherms for methane, ethane, propane, butane, and pentane for Example 2.
**FIG. 10** shows the sorption isotherms for methane, ethane, propane, butane, and pentane for Example 3.

### DETAILED DESCRIPTION OF THE PREFERRED EMBODIMENTS

Raw natural gas comes from three types of wells: oil wells, gas wells, and condensate wells. Natural gas that comes from oil wells is typically termed "associated gas". This gas can exist separate from oil in the formation (free gas), or dissolved in the crude oil (dissolved gas). Natural gas from gas and condensate wells, in which there is little or no crude oil, is termed "non-associated gas". Gas wells typically produce raw natural gas by itself, while condensate wells produce free natural gas along with a semi-liquid hydrocarbon condensate. Whatever the source of the natural gas, once separated from crude oil (if present) it commonly exists as methane in mixtures with other hydrocarbons; principally ethane, propane, butane, and pentanes and to a lesser extent heavier hydrocarbons.

Raw natural gas often contain a significant amount of impurities, such as water or acid gases, for example carbon dioxide (CO₂), hydrogen sulfide (H2S), sulfur dioxide (SO₂), carbon disulfide (CS₂), hydrogen cyanide (HCN), carbonyl sulfide (COS), or mercaptans as impurities. The term "natural gas feedstream" as used in the method of the present invention includes any natural gas source, raw or raw natural gas that has been treated one or more times to remove water and/or other impurities.

The terms "natural gas liquids" (NGL) and "ethane plus" (C₂+) refer broadly to hydrocarbons having two or more carbons such as ethane, propane, butane, and possibly small quantities of pentanes or heavier hydrocarbons. Preferably, NGL have a methane concentration of 5 mol percent or less.

The term "methane-rich" refers broadly to any vapor or liquid stream, e.g., after fractionation from which at least some ethane plus amounts have been recovered. Thus, a methane-rich stream has a higher concentration of C₁ than the concentration of C₁ in associated and non-associated natural gas. Preferably, the concentration increase of C₁ is from removal of at least 90 mole percent of the ethane in the natural and removal of at least 95 mole percent of the propane plus.

The present invention is a method to remove some or all natural gas liquids (NGLs) from natural gas, such as raw natural gas, produced in remote locations prior to flaring. The present invention provides several benefits. First, removing NGLs from natural gas prior to flaring will reduce the environmental impact of flaring the natural gas by reducing the amount of gas being burned. Further, the higher BTU value of NGLs increases the flame temperature of a flared natural gas stream. Higher temperature produces more nitrous oxides (NOₓ) so by removing some or all of the NGLs, NOₓ emissions will be lower. Additionally, NGLs are valued building blocks by chemical makers and have significantly higher value than natural gas or methane itself. Recovered NGLs can be condensed, stored as necessary, and transported by truck, rail, or other means thus capturing value that otherwise would be lost flaring them as components of the natural gas.

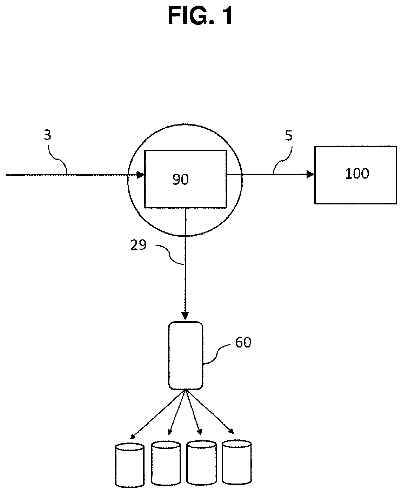

FIG. 1 shows a schematic of one embodiment of the present invention wherein raw natural gas 3 from an oil well, a gas well, or a condensate well is passed through a separation unit 90 to remove some or all of the NGLs 29 forming a methane-rich natural gas stream 5 prior to flaring 100. The NGLs are discharged 29 from the separation unit 90 and recovered individually or as a mixture of gases (e.g., as C₂, C₃, C₄, C₅, etc.) or liquefied by a means **60**, and recovered individually or as a mixture of liquids.

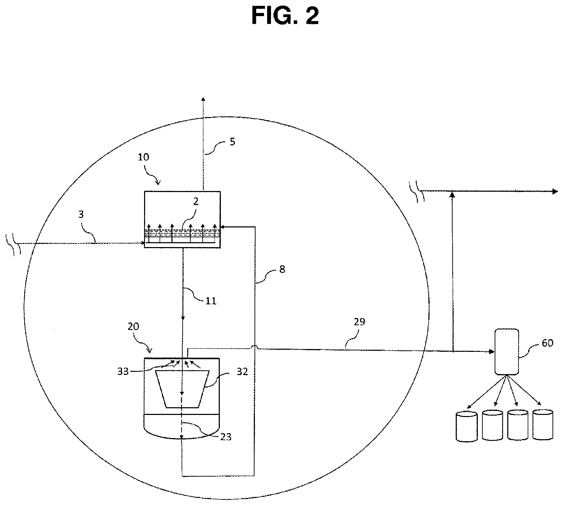

**FIG. 2** shows a schematic of the separation unit **90** for the process depicted in **FIG. 1****.** The separation unit **90** comprises an adsorption unit **10** comprising an adsorption bed **2** comprising an adsorbent media which adsorbs NGLs to form a loaded adsorbent media and a regeneration unit **20** comprising a means to regenerate loaded adsorbent media **32** causing the release of adsorbed NGLs **33** from the loaded adsorbing media and forming regenerated adsorbent media which can be transported back **8** to the adsorption unit **10** for reuse.

The separation means **90** comprises an adsorbent. Suitable adsorbents are solids having a microscopic structure. The internal surface of such adsorbents is preferably between 100 to 2000 m²/g, more preferably between 500 to 1500 m²/g, and even more preferably 1000 to 1300 m²/g. The nature of the internal surface of the adsorbent in the adsorbent bed is such that C₂ and heavier hydrocarbons are adsorbed. The adsorbent media is a porous cross-linked polymeric adsorbent or a partially pyrolized macroporous polymer. Preferably, the internal surface of the adsorbent is non-polar.

The method of the present invention makes use of an adsorbent media to extract NGLs from a natural gas stream. The mechanism by which the macroporous polymeric adsorbent extracts the NGLs from the natural gas stream is a combination of adsorption and absorption; the dominating mechanism at least is believed to be adsorption. Accordingly, the terms "adsorption" and "adsorbent" are used throughout this specification, although this is done primarily for convenience. The invention is not considered to be limited to any particular mechanism.

When an adsorbent media has adsorbed any amount of C₂+ hydrocarbons it is referred to as "loaded". Loaded includes a range of adsorbance from a low level of hydrocarbons up to and including saturation with adsorbed hydrocarbons.

The term "macroporous" is used in the art interchangeably with "macroreticular," and refers in general to pores with diameters of about 500 Å or greater. "Mesopores" are characterized as pores of between 50 Å and larger but less than 500 Å. "Micropores" are characterized as pores of less than 50 Å. The engineered distribution of these types of pores gives rise to the desired properties of high adsorption capacity for NGLs and ease of desorption of NGLs under convenient/practical chemical engineering process modifications (increase in temperature or reduced pressure [vacuum]). The process giving rise to the distribution of micropores, mesopores and macropores can be achieved in various ways, including forming the polymer in the presence of an inert diluent or other porogen to cause phase separation and formation of micropores by post cross-linking.

In one embodiment, the adsorbent media used in the method of the present invention is a macroporous polymeric adsorbent which is a post cross-linked polymeric synthetic adsorbent engineered to have high surface area, high pore volume and high adsorption capacities as well as an engineered distribution of macropores, mesopores and micropores.

Preferably, the macroporous polymeric adsorbent used in the present invention is hypercrosslinked and/or methylene bridged having the following characteristics: a BET surface area of equal to or greater than 500 m²/g and preferably equal to or greater than 1,000 m²/g, and having a particle size of 300 microns to 1500 microns, preferably 500 to 1200 microns.

Examples of monomers that can be polymerized to form macroporous polymeric adsorbents useful are styrene, alkylstyrenes, halostyrenes, haloalkylstyrenes, vinylphenols, vinylbenzyl alcohols, vinylbenzyl halides, and vinylnaphthalenes. Included among the substituted styrenes are ortho-, meta-, and para-substituted compounds. Specific examples are styrene, vinyltoluene, ethylstyrene, t-butylstyrene, and vinyl benzyl chloride, including ortho-, meta-, and para-isomers of any such monomer whose molecular structure permits this type of isomerization. Further examples of monomers are polyfunctional compounds. One preferred class is polyvinylidene compounds, examples of which are divinylbenzene, trivinylbenzene, ethylene glycol dimethacrylate, divinylsulfide and divinylpyridine. Preferred polyvinylidene compounds are di- and trivinyl aromatic compounds. Polyfunctional compounds can also be used as crosslinkers for the monomers of the first group.

One preferred method of preparing the polymeric adsorbent is by swelling the polymer with a swelling agent, then crosslinking the polymer in the swollen state, either as the sole crosslinking reaction or as in addition to crosslinking performed prior to swelling. When a swelling agent is used, any pre-swelling crosslinking reaction will be performed with sufficient crosslinker to cause the polymer to swell when contacted with the swelling agent rather than to dissolve in the agent. The degree of crosslinking, regardless of the stage at which it is performed, will also affect the porosity of the polymer, and can be varied to achieve a particular porosity. Given these variations, the proportion of crosslinker can vary widely, and is not restricted to particular ranges. Accordingly, the crosslinker can range from about 0.25% of the polymer to about 45%. Best results are generally obtained with about 0.75% to about 8% crosslinker relative to the polymer, the remaining (noncrosslinking) monomer constituting from about 92% to about 99.25% (all percentages are by weight).

Other macroporous polymeric adsorbents useful in the practice of this invention are copolymers of one or more monoaromatic monomers with one or more nonaromatic monovinylidene monomers. Examples of the latter are methyl acrylate, methyl methacrylate and methylethyl acrylate. When present, these nonaromatic monomers preferably constitute less than about 30% by weight of the copolymer.

The macroporous polymeric adsorbent is prepared by conventional techniques, examples of which are disclosed in various United States patents. Examples are USP 4,297,220; 4,382,124; 4,564,644; 5,079,274; 5,288,307; 4,950,332; and 4,965,083.

For polymers that are swollen and then crosslinked in the swollen state, the crosslinking subsequent to swelling can be achieved in a variety of ways, which are further disclosed in the patents cited above. One method is to first haloalkylate the polymer, then swell it and crosslink by reacting the haloalkyl moieties with aromatic groups on neighboring chains to form an alkyl bridge. Haloalkylation is achieved by conventional means, an example of which is to first swell the polymer under non-reactive conditions with the haloalkylating agent while including a Friedel-Crafts catalyst dissolved in the haloalkylating agent. Once the polymer is swollen, the temperature is raised to a reactive level and maintained until the desired degree of haloalkylation has occurred. Examples of haloalkylating agents are chloromethyl methyl ether, bromomethyl methyl ether, and a mixture of formaldehyde and hydrochloric acid. After haloalkylation, the polymer is swelled further by contact with an inert swelling agent. Examples are dichloroethane, chlorobenzene, dichlorobenzene, ethylene dichloride, methylene chloride, propylene dichloride, and nitrobenzene. A Friedel-Crafts catalyst can be dissolved in the swelling agent as well, since the catalyst will be used in the subsequent crosslinking reaction. The temperature is then raised to a level ranging from about 60°C to about 85°C in the presence of the catalyst, and the bridging reaction proceeds. Once the bridging reaction is complete, the swelling agent is removed by solvent extraction, washing, drying, or a combination of these procedures.

The pore size distribution and related properties of the finished adsorbent can vary widely and no particular ranges are critical to the invention. In most applications, best results will be obtained at a porosity (total pore volume) within the range of from about 0.5 to about 1.5 cc/g of the polymer. A preferred range is about 0.7 to about 1.3 cc/g. Within these ranges, the amount contributed by macropores (i.e., pores having diameters of 500 Å or greater) will preferably range from about 0.025 to about 0.6 cc/g, and most preferably from about 0.04 to about 0.5 cc/g. The surface area of the polymer, as measured by nitrogen adsorption methods such as the well-known BET method, will in most applications be within the range of about 150 to about 2100 m²/g, and preferably from about 400 to about 1400 m²/g. The average pore diameter will most often range from about 10 Å to about 100 Å.

The form of the macroporous polymeric adsorbent is likewise not critical and can be any form which is capable of containment and contact with a flowing compressed air stream. Granular particles and beads are preferred, ranging in size from about 50 to about 5,000 microns, with a range of about 500 to about 3,000 microns particularly preferred. Contact with the adsorbent can be achieved by conventional flow configurations of the gas, such as those typically used in fluidized beds or packed beds. The adsorbent can also be enclosed in a cartridge for easy removal and replacement and a more controlled gas flow path such as radial flow.

The macroporous polymeric adsorbent can function effectively under a wide range of operating conditions. The temperature will preferably be within any range which does not cause further condensation of vapors or any change in physical or chemical form of the adsorbent. Preferred operating temperatures are within the range of from 5°C to 75°C, and most preferably from 10°C to 50°C. In general, operation at ambient temperature or between ambient temperature and 10°C to 15°C above ambient will provide satisfactory results. The pressure of the natural gas stream entering the adsorbent bed can vary widely as well, preferably extending from 2 psig (115 kPa) to 1000 psig (7000 kPa). The pressure will generally be dictated by the plant unit where the product gas will be used. A typical pressure range is from 100 psig (795 kPa) to 300 psig (2170 kPa). The residence time of the natural gas stream in the adsorbent bed will most often range from 0.02 second to 5 seconds, and preferably from 0.3 second to 3.0 seconds. The space velocity of the natural gas stream through the bed will most often fall within the range of 0.03 metres (0.1 foot) per second to 1.52 metres (5 feet) per second, with a range of 0.09 metres (0.3 foot) per second to 0.91 metres (3 feet) per second preferred. Finally, the relative humidity can have any value up to 100%, although for convenience, the preferred range of relative humidity is about 25% to about 98%.

The macroporous polymeric adsorbents used in the present invention described herein above can be used to separate ethane, propane, butane, pentane, and heaver hydrocarbons from mixed gases containing methane. Preferably, the macroporous polymeric adsorbents used in the present invention adsorb equal to or greater than 60 cm³ STP of propane per gram of sorbent at 35°C and 500 mmHg of propane. Preferably, the adsorbents used in the present invention adsorb equal to or greater than 60 cm³ STP of n-butane per gram of sorbent at 35°C and 100 mmHg of n-butane. Furthermore, these materials are able to be degassed of propane or n-butane and then able to readsorb equal to or greater than 60 cm³ STP of propane per gram of sorbent at 35°C and 500 mmHg of propane or readsorb greater than 60 cm³ STP of n-butane per gram of sorbent at 35°C and 100 mmHg of n-butane at least once. Preferably, the adsorbents used in the present invention adsorb equal to or greater than 30 cm³ STP of ethane per gram of sorbent at 35°C and 600 mmHg of ethane. Preferably, the adsorbents used in the present invention adsorb equal to or greater than 100 cm³ STP of pentane per gram of sorbent at 35°C and 50 mmHg of pentane.

In another embodiment, the adsorbent media used in the present invention is a partially pyrolized macroporous polymeric adsorbent media to extract NGLs from a natural gas stream.

Pyrolized macroporous polymeric adsorbent media are well known, for instance see USP 4,040,990. Partially pyrolyzed particles, preferably in the form of beads or spheres, produced by the controlled decomposition of a synthetic polymer of specific initial porosity. In a preferred embodiment, the pyrolyzed particles are derived from the thermal decomposition of macroreticular ion exchange resins containing a macroporous structure.

In general pyrolysis comprises subjecting the starting polymer to controlled temperatures for controlled periods of time under certain ambient conditions. The primary purpose of pyrolysis is thermal degradation while efficiently removing the volatile products produced.

The maximum temperatures may range from about 300°C to up to about 900°C, depending on the polymer to be treated and the desired composition of the final pyrolyzed particles. Higher temperature, e.g., about 700°C and higher result in extensive degradation of the polymer with the formation of molecular sieve sized pores in the product.

Most desirably, thermal decomposition (alternatively denoted "pyrolysis" or "heat treatment") is conducted in an inert atmosphere comprised of, for example, argon, neon, helium, nitrogen, or the like, using beads of macroreticular synthetic polymer substituted with a carbon-fixing moiety which permits the polymer to char without fusing in order to retain the macroreticular structure and give a high yield of carbon. Among the suitable carbon-fixing moieties are sulfonate, carboxyl, amine, halogen, oxygen, sulfonate salts, carboxylate salts and quaternary amine salts. These groups are introduced into the starting polymer by well-known conventional techniques, such as those reactions used to functionalize polymers for production of ion exchange resins. Carbon-fixing moieties may also be produced by imbibing a reactive precursor thereof into the pores of macroreticular polymer which thereupon, or during heating, chemically binds carbon-fixing moieties onto the polymer. Examples of these latter reactive precursors include sulfuric acid, oxidizing agents, nitric acid, Lewis acids, acrylic acid, and the like.

Suitable temperatures for practicing the process of pyrolysis are generally within the range of 300°C to about 900°C, although higher temperatures may be suitable depending upon the polymer to be treated and the desired composition of the final pyrolyzed product. At temperatures above about 700°C the starting polymer degrades extensively with the formation of molecular sieve sized pores in the product, i.e., 4 Å to 6 Å average critical dimension, yielding a preferred class of adsorbents for use in the method of the invention. At lower temperatures, the thermally-formed pores usually range from 6 Å to as high as 50 Å in average critical size. A preferred range of pyrolysis temperatures is between about 400°C and 800°C. As will be explained more fully hereinafter, temperature control is essential to yield a partially pyrolyzed material having the composition, surface area, pore structures and other physical characteristics of the desired product. The duration of thermal treatment is relatively unimportant, providing a minimum exposure time to the elevated temperature is allowed.

A wide range of pyrolyzed resins may be produced by varying the porosity and/or chemical composition of the starting polymer and also by varying the conditions of thermal decomposition. In general, the partially pyrolyzed resins used in the invention have a carbon to hydrogen ratio of 1.5 : 1 to 20 : 1, preferably 2.0 : 1 to 10 : 1, whereas activated carbon normally has a C/H ratio much higher, at least greater than 30 : 1 (Carbon and Graphite Handbook, Charles L. Mantell, Interscience Publishers, N.Y. 1968, p. 198). The product particles contain at least 85% by weight of carbon with the remainder being principally hydrogen, alkali metals, alkaline earth metals, nitrogen, oxygen, sulfur, chlorine, etc., derived from the polymer or the functional group (carbon-fixing moiety) contained thereon and hydrogen, oxygen, sulfur, nitrogen, alkali metals, transition metals, alkaline earth metals and other elements introduced into the polymer pores as components of a filler (may serve as a catalyst and/or carbon-fixing moiety or have some other functional purpose).

The pore structure of the final product must contain at least two distinct sets of pores of differing average size, i.e., multimodal pore distribution. The larger pores originate from the macroporous resinous starting material which preferably contain macropores ranging from between 50 Å to 100,000 Å in average critical dimension. The smaller pores, as mentioned previously, generally range in size from 4 Å to 50 Å, depending largely upon the maximum temperature during pyrolysis. Such multimodal pore distribution is considered a novel and essential characteristic of the composition used in the invention.

The partially pyrolyzed polymers used in the invention have relatively large surface area resulting from the macroporosity of the starting material and the smaller pores developed during pyrolysis. In general the overall surface area as measured by nitrogen adsorption ranges between about 50 and 1500 m²/gram. Of this, the macropores will normally contribute 6 to 700 m²/gram, preferably 6 to 200 m²/g, as calculated by mercury intrusion techniques, with the remainder contributed by the thermal treatment. Pore-free polymers, such as "gel" type resins which have been subjected to thermal treatment in the prior art do not contribute the large pores essential to the adsorbents used in the invention nor do they perform with the efficiency of the pyrolyzed polymers described herein.

The duration of pyrolysis depends upon the time needed to remove the volatiles from the particular polymer and the heat transfer characteristics of the method selected. In general, the pyrolysis is very rapid when the heat transfer is rapid, e.g., in an oven where a shallow bed of material is pyrolyzed, or in a fluidized bed. To prevent burning of the pyrolyzed polymer, normally the temperature of the polymer is reduced to not more than 400°C, preferably not more than 300°C, before the pyrolyzed material is exposed to air. The most desirable method of operation involves rapid heating to the maximum temperature, holding the temperature at the maximum for a short period of time (in the order of 0 to 20 minutes) and thereafter quickly reducing the temperature to room temperature before exposing the sample to air. Products for use in the method of the invention have been produced by this preferred method by heating to 800°C and cooling in a period of 20 to 30 minutes. Longer holding periods at the elevated temperatures are also satisfactory, since no additional decomposition appears to occur unless the temperature is increased.

Activating gases such as CO₂, NH₃, O₂, H₂O or combinations thereof in small amounts tend to react with the polymer during pyrolysis and thereby increase the surface area of the final material. Such gases are optional and may be used to obtain special characteristics of the adsorbents.

The starting polymers which may be used to produce the pyrolyzed resins for use in the method of the invention include macroreticular homopolymers or copolymers of one or more monoethylenically or polyethylenically unsaturated monomers or monomers which may be reacted by condensation to yield macroreticular polymers and copolymers. The macroreticular resins used as precursors in the formation of macroreticular heat treated polymers are not claimed as new compositions of matter in themselves. Any of the known materials of this type with an appropriate carbon-fixing moiety is suitable. The preferred monomers are those aliphatic and aromatic materials which are ethylenically unsaturated.

Examples of suitable monoethylenically unsaturated monomers that may be used in making the granular macroreticular resin include: esters of acrylic and methacrylic acid such as methyl, ethyl, 2-chloro ethyl, propyl, isobutyl, isopropyl, butyl, tert-butyl, sec-butyl, ethylhexyl, amyl, hexyl, octyl, decyl, dodecyl, cyclohexyl, isobornyl, benzyl, phenyl, alkylphenyl, ethoxymethyl, ethoxyethyl, ethoxypropyl, propoxymethyl, propoxyethyl, propoxypropyl, ethoxyphenyl, ethoxybenzyl, ethoxycyclohexul, hydroxyethyl, hydroxypropyl, ethylene, propylene, isobutylene, diisobutylene, styrene, ethylvinylbenzene, vinyltoluene, vinylbenzylchloride, vinyl chloride, vinyl acetate, vinylidene chloride, dicyclopentadiene, acrylonitrile, methacrylonitrile, acrylamide, methacrylamide, diacetone acrylamide, functional monomers such as vinylbenzene, sulfonic acid, vinyl esters, including vinyl acetate, vinyl propionate, vinyl butyrate, vinyl laurate, vinyl ketones including vinyl methyl ketone, vinyl ethyl ketone, vinyl isopropyl ketone, vinyl n-butyl ketone, vinyl hexyl ketone, vinyl octyl ketone, methyl isopropenyl ketone, vinyl aldehydes including acrolein, methacrolein, crotonaldehyde, vinyl ethers including vinyl methyl ether, vinyl ethyl ether, vinyl propyl ether, vinyl isobutyl ether, vinylidene compounds including vinylidene chloride bromide, or bromochloride, also the corresponding neutral or half-acid half-esters or free diacids of the unsaturated dicarboxylic acids including itaconic, citraconic, aconitic, fumaric, and maleic acids, substituted acrylamides, such as N-monoalkyl, -N,N-dialkyl-, and N-dialkylaminoalkylacrylamides or methacrylamides where the alkyl groups may have from one to eighteen carbon atoms, such as methyl, ethyl, isopropyl, butyl, hexyl, cyclohexyl, octyl, dodecyl, hexadecyl and octadecyl aminoalkyl esters of acrylic or methacrylic acid, such as .beta.-dimethylaminoethyl, .beta.-diethylaminoethyl or 6-dimethylaminohexyl acrylates and methacrylates, alkylthioethyl methacrylates and acrylates such as ethylthioethyl methacrylate, vinylpyridines, such as 2-vinylpyridine, 4-vinylpyridine, 2-methyl-5-vinylpyridine, and so on.

In the case of copolymers containing ethylthioethyl methacrylate, the products can be oxidized to, if desired, the corresponding sulfoxide or sulfone.

Polyethylenically unsaturated monomers which ordinarily act as though they have only one such unsaturated group, such as isoprene, butadiene, and chloroprene, may be used as part of the monoethylenically unsaturated category.

Examples of polyethylenically unsaturated compounds include: divinylbenzene, divinylpyridine, divinylnaphthalenes, diallyl phthalate, ethylene glycol diacrylate, ethylene glycol dimethacrylate, trimethylolpropanetrimethacrylate, divinylsulfone, polyvinyl or polyallyl ethers of glycol, of glycerol, of pentaerythritol, of diethyleneglycol, of monothio or dithio-derivatives of glycols, and of resorcinol, divinylketone, divinylsylfide, allyl acrylate, diallyl maleate, diallyl fumarate, diallyl succinate, diallyl carbonate, diallyl malonate, diallyl oxalate, diallyl adipate, diallyl sebacate, divinyl sebacate, diallyl tartrate, diallyl silicate, triallyl tricarballylate, triallyl aconitate, triallyl citrate, triallyl phosphate, N,N'-methylenediacrylamide, N,N'-methylenedimethacrylamide, N,N'-ethylenediacrylamide, trivinylbenzene, trivinylnaphthalenes, and polyvinylanthracenes.

A preferred class of monomers of this type is aromatic ethylenically unsaturated molecules such as styrene, vinyl pyridine, vinyl naphthalene, vinyl toluene, phenyl acrylate, vinyl xylenes, and ethylvinylbenzene.

Examples of preferred polyethylenically unsaturated compounds include divinyl pyridine, divinyl naphthalene, divinylbenzene, trivinylbenzene, alkyldivinylbenzenes having from 1 to 4 alkyl groups of 1 to 2 carbon atoms substituted in the benzene nucleus, and alkyltrivinylbenzenes having 1 to 3 alkyl groups of 1 to 2 carbon atoms substituted in the benzene nucleus. Besides the homopolymers and copolymers of these poly(vinyl) benzene monomers, one or more of them may be copolymerized with up to 98% (by weight of the total monomer mixture) of (1) monoethylenically unsaturated monomers, or (2) polyethylenically unsaturated monomers other than the poly(vinyl)benzenes just defined, or (3) a mixture of (1) and (2). Examples of the alkyl-substituted di- and tri-vinyl-benzenes are the various vinyltoluenes, the divinylethylbenzene, 1,4-divinyl- 2,3,5,6-tetramethylbenzene, 1,3,5-trivinyl-2,4,6-trimethylbenzene, 1,4-divinyl, 2,3,6-triethylbenzene, 1,2,4-trivinyl-3,5-diethylbenzene, 1,3,5-trivinyl-2-methylbenzene.

Most preferred are copolymers of styrene, divinylbenzene, and ethylvinylbenzene.

Examples of suitable condensation monomers include: (a) aliphatic dibasic acids such as maleic acid, fumaric acid, itaconic acid, 1,1-cyclobutanedicarboxylic acid, etc.; (b) aliphatic diamines such as piperazine, 2-methylpiperazine, cis, cis-bis (4-aminocyclohexyl) methane, metaxylylenediamine, etc.; (c) glycols such as diethylene glycol, triethylene glycol, 1,2-butanediol, neopentyl glycol etc.; (d) bischloroformates such as cis and trans- 1,4-cyclohexyl bischloroformate, 2,2,2,4-tetramethyl-1,3-cyclobutyl bischloroformate and bischloroformates of other glycols mentioned above, etc.; (e) hydroxy acids such as salicylic acid, m- and p-hydroxybenzoic acid and lactones, derived therefrom such as the propiolactones, valerolactones, caprolactones, etc.; (f) diisocyanates such as cis and trans-cyclopropane-1,2 -diisocyanate, cis and trans-cyclobutane-1-2-diisocyanate etc.; (g) aromatic diacids and their derivatives (the esters, anhydrides and acid chlorides) such as phthalic acid, phthalic anhydride, terephthalic acid, isophthalic acid, dimethylphthalate, etc.; (h) aromatic diamines such as benzidine, 4,4'-methylenediamine, bis(4-aminophenyl) ether, etc.; (i) bisphenols such as bisphenol A, bisphenol C, bisphenol F, phenolphthalein, recorcinol, etc.; (j) bisphenol bis(chloroformates) such as bisphenol A bis(chloroformate), 4,4' -dihydroxybenzophenone bis(chloroformate) etc.; (k) carbonyl and thiocarbonyl compounds such as formaldehyde, acetaldehyde, thioacetone acetone, etc.; (1) phenol and derivatives such as phenol, alkylphenols, etc.; (m) polyfunctional cross-linking agents such as tri or poly basic acids such as trimellitic acid, tri or polyols such as glycerol, tri or polyamines such as diethylenetriamine; and other condensation monomers and mixtures of the foregoing.

Ion exchange resins produced from aromatic and/or aliphatic monomers provide a preferred class of starting polymers for production of porous adsorbents. The ion exchange resin may also contain a functional group selected from cation, anion, strong base, weak base, sulfonic acid, carboxylic acid, oxygen containing, halogen and mixtures of the same. Further, such ion exchange resins may optionally contain an oxidizing agent, a reactive substance, sulfuric acid, nitric acid, acrylic acid, or the like at least partially filling the macropores of the polymer before heat treatment.

The synthetic polymer may be impregnated with a filler such as carbon black, charcoal, bonechar, sawdust or other carbonaceous material prior to pyrolysis. Such fillers provide an economical source of carbon which may be added in amounts up to about 90% by weight of the polymer.

The starting polymers, when ion exchange resins, may optionally contain a variety of metals in their atomically dispersed form at the ionic sites. These metals may include iron, copper, silver, nickel, manganese, palladium, cobalt, titanium, zirconium, sodium, potassium, calcium, zinc, cadmium, ruthenium, uranium and rare earths such as lanthanum. By utilizing the ion exchange mechanism it is possible for the skilled technician to control the amount of metal that is to be incorporated as well as the distribution.

Although the incorporation of metals onto the resins is primarily to aid their ability to serve as catalytic agents, useful adsorbents may also contain metal.

Synthetic polymers, ion exchange resins whether in the acid, base or metal salt form are commercially available.

The partially pyrolyzed macroporous polymer adsorbent used in the present invention disclosed herein above are able to adsorb greater than 25 cm³ STP of ethane per gram of sorbent at 35°C and 200 mmHg of ethane and greater than 30 cm³ STP of propane per gram of sorbent at 35°C and 100 mmHg of propane. Furthermore, these materials are able to be degassed of ethane or propane and then able to readsorb greater than 25 cm³ STP of ethane per gram of sorbent at 35°C and 200 mmHg of ethane, or readsorb greater than 30 cm³ STP of propane per gram of sorbent at 35°C and 100 mmHg of propane one or more times.

The separation process of the invention comprises passing a natural gas stream through an adsorber bed charged with the adsorbent(s) described. Preferably, the ethane and/or propane and/or butane and/or pentane and/or heavier hydrocarbons, which are selectively adsorbed, can be readily desorbed either by lowering the pressure or by increasing the temperature of the adsorber bed resulting in a regenerated adsorbent. The adsorbent so regenerated can be reused as an adsorbent for the separation of ethane and/or propane and/or butane and/or pentane and/or heavier hydrocarbons from the natural gas stream.

Batch, semi-continuous, and continuous processes and apparatuses for separating NGLs from natural gas feedstreams are well known. **FIG. 2** depicts one embodiment of a separation process of the present invention. The separation unit **90** comprises an adsorption unit **10** and a regeneration unit **20.** The separation process comprises the steps of (a) passing a natural gas feedstream 3 through an adsorption unit **10** comprising an adsorbent bed **2** comprising an adsorbent media which adsorbs heavier hydrocarbons (C₂, C₃, C₄, C₅, etc.) to obtain a methane-rich natural gas product **5** which is then flared **100,** (b) transporting **11** adsorbent loaded with heavier hydrocarbons from the adsorption unit **10** to a regeneration unit **20** comprising a means **32** to regenerate the loaded adsorbent media whereby by causing the release of the heavier hydrocarbons **33** from the loaded adsorbing media and forming regenerated adsorbent media **23,** (c) wherein the regenerated adsorbent media **23** is transported **8** back to the adsorption unit **10** for reuse, and (d) the released heavier hydrocarbons **33** are discharged **29,** to be recovered either individually or as a mixture of gases (e.g., as C₂, C₃, C₄, C₅, etc.) or liquefied by a means **60,** and recovered either individually or as a mixture of liquids.

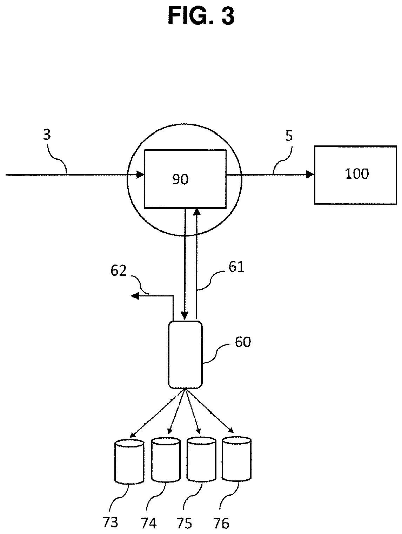

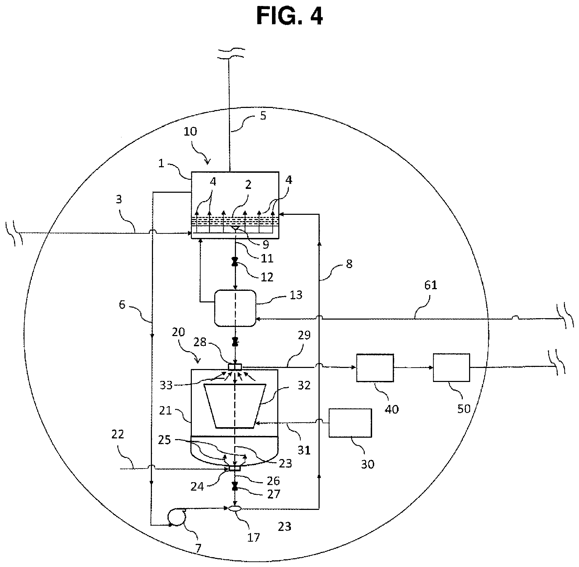

Although a particular preferred embodiment of the invention is disclosed in **FIGs. 3** and **4** for illustrative purposes, it will be recognized that variations or modifications of the disclosed process lie within the scope of the present invention.

The adsorption step and/or the regeneration step of the process of the present invention may operate in as a batch process, a semi-continuous process, a continuous process, or combination thereof. For instance in one embodiment of the present invention, both the adsorption step and the regeneration step may operate in the batch mode. In another embodiment of the present invention both the adsorption step and the regeneration step may operate in the semi-continuous mode. In yet another embodiment of the present invention both the adsorption step and the regeneration step may operate in the continuous mode.

Alternatively, in one embodiment of the present invention the adsorption step may operate in a batch, semi-continuous, or continuous mode while the regeneration step operates in a different mode than that of the adsorption step. For example, in one embodiment of the present invention the adsorption step may operate in a batch mode while the regeneration step operates in a continuous mode. In another embodiment of the present invention the adsorption step may operate in a continuous mode while the regeneration step operates in a continuous mode. All possible combinations of batch, semi-continuous, and continuous modes for the adsorbent step and regeneration step are considered within the scope of the present invention.

Adsorption is in many situations a reversible process. The practice of removing volatiles from an adsorption media can be accomplished by reducing the pressure over the media, heating, or the combination of reduced pressure and heating. In either case the desired outcome is to re-volatilize the trapped vapors, and subsequently remove them from the adsorbent so that it can be reused to capture additional volatiles. Preferably, the adsorption media of the present invention when regenerated, desorbs adsorbed gases in an amount equal to or greater than 75 percent of the amount adsorbed, more preferably equal to or greater than 85 percent, more preferably equal to or greater than 90 percent, more preferably equal to or greater than 95 percent, more preferably equal to or greater than 99 percent and most preferably virtually all the NGLs adsorbed.

Traditional means of heating adsorbent media for the purpose of removing adsorbed volatiles that utilize conventional heating systems such as heated gas (air or inert gas), or radiant heat contact exchangers are suitable for use in the present NGL separation process as part of the adsorbent media regeneration step.

Preferably, the NGL separation process of the present invention employs a microwave heating system as part of the adsorbent media regeneration step. Such a microwave heating system provides a heating system and method for removing volatiles from adsorbent media with higher thermal efficiency at a reduced cost.

Referring to **FIGs. 3** and **4****,** another embodiment of the method of the present invention is shown wherein a NGL separation unit **90** comprises an adsorption unit **10** which has an adsorption tank **1** containing an adsorbent bed **2** comprising the adsorption media described herein. The natural gas feedstream enters the adsorption unit **10** via line **3** at the lower portion of the adsorption tank **1** and passes **4** through the adsorbent bed **2.** The adsorption bed **2** comprises an adsorbent media which can adsorb C₂, C₃, C₄, C₅, and heavier hydrocarbons from the natural gas feedstream. Inlet temperature of the adsorption unit **10** can range from 5 to 100°C, preferably from 15 to 80°C, and more preferably from 20 to 70°C. Pressures of 96 to 9653 kPa (14 to 1400 psia), preferably from 4137 to 8274 kPa (600 to 1200 psia), and more preferably from 5516 to 6895 kPa (800 to 1000) psia can be used. A methane-rich natural gas product stream a vastly reduced heavy hydrocarbon content than natural gas feedstream leaves the adsorbent bed **2** and is leaves from the top of the adsorption tank **1** through line **5.** The methane-rich natural gas stream **5** is transported to be flared **100.**

As seen in **FIG. 4****,** as the adsorption media becomes loaded with NGLs it passes through the bottom of the adsorption tank **1** through a transport mechanism **9** through line **11** into a microwave regeneration unit **20** having a regeneration tank **21** and a microwave heating system **32.** The operating temperatures of the microwave heating system **32** can range from 105 to 350°C, preferably from 140 to 250°C, and more preferably from 145 to 200°C. Pressures of from 138 to 4137 kPa (20 to 600 psia), preferably 689 to 2758 kPa (100 to 400 psia), and more preferably 1034 to 1379 kPa (150 to 200 psia) can be used. The microwave power source **30** heats the adsorbent media **2** in the microwave heating system **32** causing the NGLs to vaporize **33.**

The microwave heating system **32** can irradiate a loaded adsorbent media to desorb volatile materials. Irradiation of adsorbent media with microwave radiation can provide an economical and thermally efficient alternative for heating adsorbent materials to remove adsorbed volatiles from the adsorbent. Microwave radiation energy can be applied to an adsorbent without heating a gas, and can effectively transfer thermal energy to specific adsorbents through path lengths in excess of 0.30 metres (12 inches). To accomplish this method of heating the adsorbent media, the apparatus for applying or generating the microwave radiation for a heating device must be constructed in such a manner as to afford uniform heating of the
adsorbent, and to minimize or eliminate any reflection of the radiation back onto the microwave power source **30.** The microwave heating system **32** can include a heating apparatus and a heating or radiation system (not shown in **FIG. 4**), and optionally a purge gas system **24.** The heating apparatus can be coupled to and in communication with the radiation system for receipt of thermal energy generated by the radiation system, such as microwave radiation or electromagnetic energy, and with the purge gas system **24** for receipt of a purge gas to assist in the removal of volatiles from the adsorbent.

The NGLs are extracted from the regeneration tank **21** through a suction port **28** via a vacuum evacuation system **40.** The regeneration tank **21** may optionally be fitted with a purge gas system **24** wherein purge gas, for example nitrogen, enters through line **22** and is dispersed **25** at the bottom of the regeneration tank **21.**

The regenerated adsorbent media **23** is allowed to pass from the bottom of the regeneration tank **21** through line **26** then returned to the adsorption tank **1.** A portion of the methane-rich natural gas from the top of the adsorber tank **1** is circulated via line **6** through blower **7** to transport the regenerated adsorption media **23** through line **8** to once again adsorb NGLs from natural gas **3.**

The NGLs vacuum extracted from the regeneration tank **21** pass through the vacuum extraction system **40** through a gas compression system **50** and introduced into a condenser **60** where the NGLs are condensed, optionally separated, and discharged either as a mixture of NGLs or individual fractions of ethane, propane, butane, pentane, and/or heavier hydrocarbons into one or more tank **73, 74, 75,** and/or **76.** The discharged NGLs may be recovered, liquefied, stored, and/or transported by truck, rail, ship, or any other convenient means. Any methane making it to the condenser is recycled back to the adsorption tank **1** through line **61** and any other gas(es), purge gas, water, and/or contaminants can be separated through line **62** to be recovered or flared along with the methane-rich natural gas stream **5.**

In one embodiment of the present invention, the NGL separation process **90** is a continuous process with continuous adsorbent media regeneration. For example, in **FIG. 4** there is a valve **12** in line **11** between the adsorber tank 1 and the regeneration tank **21** and a valve **27** in the line **26** between the regeneration tank **21** and collection tank **17.** Valves **12** and **27** are synchronized to allow for holding loaded adsorption media from the adsorption tank **1** while adsorption media is being regenerated in the regenerator unit **20.** When the adsorption media is regenerated in the regenerator tank **21,** valve **27** allows the regenerated adsorption media **23** to leave the regenerator tank **21** and be transported back to the adsorption tank **1.** Then valve **12** allows loaded adsorption media to enter the regenerator tank **21** to be regenerated. This process is repeated and allows for a continuous regeneration of the adsorption media.

In another embodiment of the present invention, the NGL separation process **90** is a batch process with batch adsorbent media regeneration. For example, in **FIG. 4** there is a holding tank **13** between the adsorption tank **1** and the regeneration tank **21.** When the adsorbent media **2** is loaded, all of it is conveyed from the adsorption tank **1** through the transport mechanism **9** and line **11** to the holding tank **13.** The contents of the holding tank **13** are then transported through line **15** to the regeneration tank **21** where the loaded adsorbent media is regenerated and returned to the adsorbent tank **1** where it is used until loaded and the process repeated.

Preferably the adsorbent used in the method of the present invention when loaded with hydrocarbons, is regenerated using a microwave regeneration system, for example as shown in **FIG. 4****.** Preferably, the microwave regeneration system is able to operate in a batch, semi-continuous, or continuous process. One advantage of using a microwave system in conjunction with adsorbents in the present invention is that it allows the microwaves to minimize the heating of the media, but maximize heating of the NGLs to encourage desorption. As such it has the benefits of being operationally simpler than traditional regeneration systems, and reduces the heat effects on the adsorbent material itself. Furthermore, when this desorption process is used in conjunction with a continuous adsorption process such as a moving packed bed or similar device, the hydrocarbon removal can be closely tailored to the composition of the feed gas such that the recovered gas can have improved purity and, when present, reduced load on the subsequent chiller apparatus which allows for recovery and later transport as a liquid.

### EXAMPLES

A description of the raw materials used in the Examples is as follows.
- Example 1: is a porous cross-linked polymeric adsorbent having a high surface area equal to or greater than 1,000 m²/g made from a macroporous copolymer of a monovinyl aromatic monomer and a crosslinking monomer, where the macroporous copolymer has been post-crosslinked in the swollen state in the presence of a Friedel-Crafts catalyst;
- Example 2: is a porous cross-linked polymeric adsorbent having a surface area equal to or greater than 1,000 m²/g made from a macroporous copolymer of a monovinyl aromatic monomer and a crosslinking monomer, where the macroporous copolymer has been post-crosslinked in the swollen state in the presence of a Friedel-Crafts catalyst with post capping of residual chloromethyl groups with hydrophobic aromatic compounds resulting in a media that has increased hydrophobicity; and
- Example 3: is a partially pyrolized macroporous polymer of a monovinyl aromatic monomer and a crosslinking monomer that has been sulfonated.

Adsorption capacity and breakthrough properties are determined for Example 1 and Example 2 as followed:

### Adsorption Capacity

### Methane, Ethane, Propane and Butane:

A Micromeritics ASAP 2020 Surface Area and Porosity Analyzer is used to analyze methane (Sigma-Aldrich, 99.0%), ethane (Sigma-Aldrich, 99.99), propane (Sigma-Aldrich, 99.97%), and butane (Matheson Tri-Gas, 99.9%) adsorption at 308 K. Prior to analysis, the macroporous polymeric adsorbent being tested (.3 to .5 grams) is degassed in a quartz U-tube at 423 K under vacuum to a pressure below 5 µmHg for 12 hours. Pressure points are taken between 5 to 600 mmHg with a 45 seconds equilibration interval. The samples are then evacuated under vacuum for 1 hour before repeating the pressure points.

### Pentane:

A Micromeritics ASAP 2020 Surface Area and Porosity Analyzer equipped with vapor introduction option with dual-zone temperature control is used to analyze static pentane adsorption at 273 K. An ethylene glycol/water mixture contained within a chiller dewer is used as temperature control for the sample. Pentane (Sigma-Aldrich, anhydrous, ≥ 99%) is placed in a quartz vessel located in the temperature-regulated vapor furnace which is controlled to 308K. Prior to pentane analysis, the macroporous polymeric adsorbent being tested is degassed in a quartz tube at 373 K under vacuum to a pressure below 5 µmHg for at least 12 hours. Relative pressure points are taken between 0.005 < P/P₀ < 0.50. The saturation pressure, P₀, was calculated to be 183.526 mmHg based on pentane adsorptive properties and the analysis bath temperature.

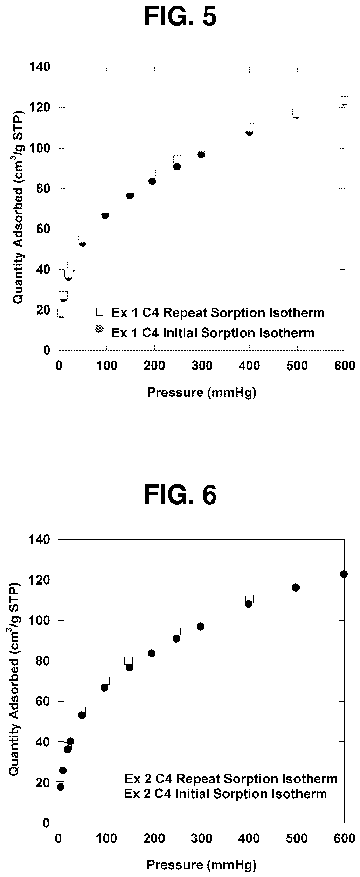

**FIGs. 5** and **6** show the initial and repeat adsorption isotherms for butane for Example 1 and Example 2, respectively.

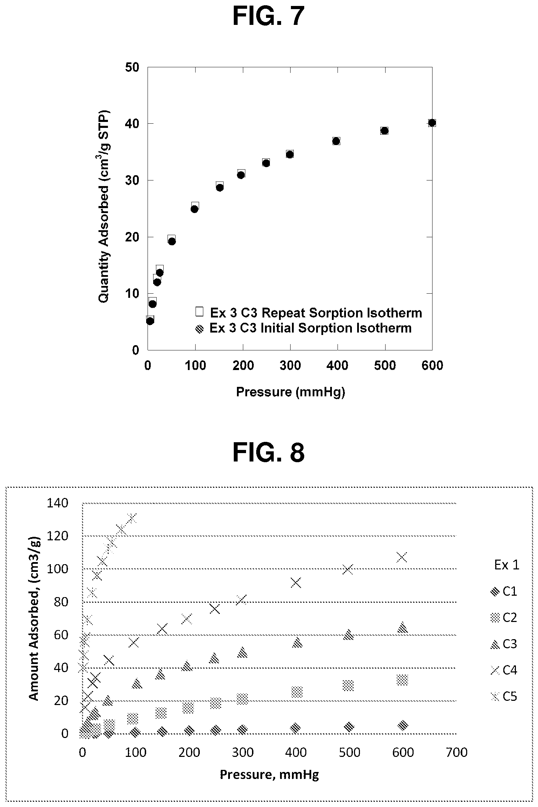

**FIG. 7** shows the initial and repeat adsorption isotherms for propane for Example 3.

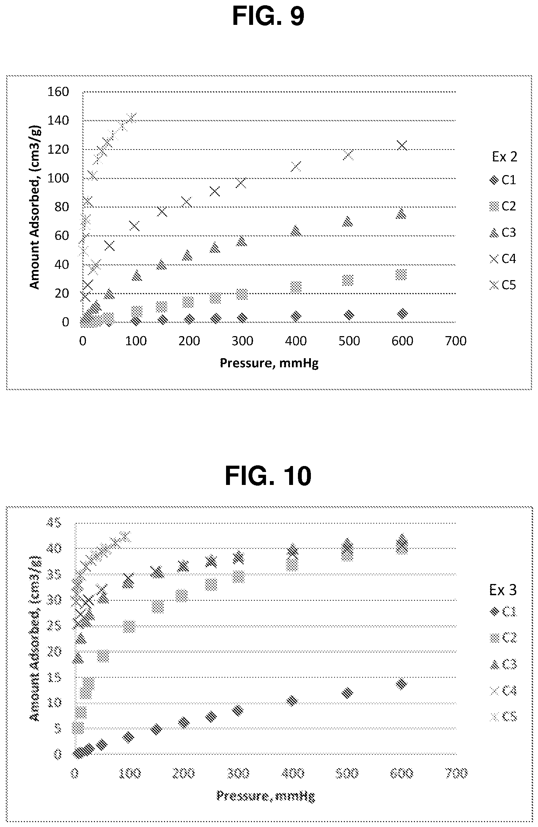

**FIGs. 8****,** **9,** and **10** show the adsorption isotherms for ethane (C2), propane (C3), butane (C4), and pentane (C5) for Examples 1, 2, and 3, respectively.

### Adsorption Breakthrough

Breakthrough curve data for the macroporous polymeric adsorbent is determined using a GC/mass spectrometer (mass spec). The GC/mass spec is calibrated then a 40g sample is loaded into the sample column. A mixed gas comprising a ratio of CH₄/C₂H₆ /C₃H₈/C4H₁₀ at 40/40/40/40 standard cubic centimeters per minute (SCCM) is analyzed. Gas flow is initiated. This flow by-passes the packed bed (i.e., column). The system is allowed to equilibrate for 2 hours. The gas from the by-pass is then analyzed by the mass spec. Following a two minute delay, the three-way valve is opened to allow the mixed gas to enter the packed bed column. The data for the mass spec analysis of the mixed gas leaving the packed bed column is recorded. The system is allowed to run until all four gases have been analyzed in the mass spec and recorded. Table 1 lists the breakthrough times for each gas.

**Table 1**

| Polymeric Sorbent Media | Example 1 | Example 2 | Example 3 |
|---|---|---|---|
| Weight, g | 40 | 40 | 40 |
| Volume, cc | 109 | 130 | 71 |
| Bulk Density, g/cc | 0.37 | 0.31 | 0.56 |
| Methane breakthrough, min | 5.2 | 6 | 6.3 |
| Ethane breakthrough, min | 13.2 | 16.5 | 11.1 |
| Propane Breakthrough, min | 27.3 | 33.2 | 16.4 |
| Butane breakthrough, min | 64 | 81.4 | 31.9 |

## Claims

1. A method for separating and recovering some or all natural gas liquids (NGLs): ethane, propane, butane, pentane, or heavier hydrocarbons from a natural gas feedstream forming a methane-rich natural gas supply and then flaring said methane-rich natural gas supply, wherein the NGLs are separated from the natural gas feedstream by means of a NGLs separation unit (90), wherein the NGLs separation unit (90) comprises:
(i) an adsorption unit (10) comprising an adsorption bed (2) comprising an adsorbent media which adsorbs NGLs to form a loaded adsorbent media and
(ii) a regeneration unit (20) comprising a means (32) to regenerate loaded adsorbent media by causing the release of adsorbed NGLs from the loaded adsorbing media and forming regenerated adsorbent media
wherein the method comprises the steps of:
(a) passing the natural gas feedstream through the adsorption unit (10) generating the adsorbent loaded with NGLs and a methane-rich natural gas supply,
(b) transporting the adsorbent loaded with NGLs from the adsorption unit (10) to the regeneration unit (20),
(c) regenerating the adsorbent loaded with NGLs by releasing the adsorbed NGLs from the loaded adsorbing media and forming regenerated adsorbent media,
(d) transporting the regenerated adsorbent media back to the adsorption unit (10) for reuse,
(e) recovering the released NGLs,
and
(f) flaring the methane-rich natural gas supply,
wherein the adsorption media is a porous cross-linked polymeric adsorbent, a partially pyrolized macroporous polymer, or mixtures thereof.

2. The method of Claim 1 wherein the natural gas feedstream is from an oil well, a gas well, or a condensate well.

3. The method of Claim 1 wherein the loaded adsorption media is regenerated by means of reduced pressure over the media, heating the media, or a combination of reduced pressure and heating.

4. The method of Claim 1 wherein the loaded adsorption media is regenerated by a microwave heating system (32).

5. The method of Claim 1 wherein the regeneration step is operated as a batch process, a semi-continuous process, or as a continuous process.

6. The method of Claim 1 wherein the recovered NGLs are stored and/or transported by truck, rail, or ship individually or as a mixture of gases (e.g., as C₂, C₃, C₄, C₅, etc.) or liquefied individually or as a mixture of liquids.

## Patentansprüche

1. Ein Verfahren zum Trennen und Wiedergewinnen eines Teils oder aller der Erdgasflüssigkeiten (NGL) Ethan, Propan, Butan, Pentan oder schwereren Kohlenwasserstoffe aus einem Erdgaszustrom, wodurch eine methanreiche Erdgaszufuhr gebildet wird, und dann Abfackeln der methanreichen Erdgaszufuhr, wobei die NGL mittels einer NGL-Trenneinheit (90) aus dem Erdgaszustrom getrennt werden, wobei die NGL-Trenneinheit (90) Folgendes beinhaltet:
(i) eine Adsorptionseinheit (10), beinhaltend ein Adsorptionsbett (2), das ein adsorbierendes Medium beinhaltet, welches NGL adsorbiert, sodass ein beladenes adsorbierendes Medium gebildet wird,
und
(ii) eine Regeneriereinheit (20), die ein Mittel (32) beinhaltet, um beladenes adsorbierendes Medium zu regenerieren, indem die Freisetzung adsorbierter NGL aus dem beladenen absorbierenden Medium verursacht wird und regeneriertes adsorbierendes Medium gebildet wird,
wobei das Verfahren die folgenden Schritte beinhaltet:
(a) Führen des Erdgaszustroms durch die Adsorptionseinheit (10), wodurch das mit NGL beladene Adsorptionsmittel und eine methanreiche Erdgaszufuhr erzeugt werden,
(b) Transportieren des mit NGL beladenen Adsorptionsmittels aus der Adsorptionseinheit (10) zu der Regeneriereinheit (20),
(c) Regenerieren des mit NGL beladenen Adsorptionsmittels durch Freisetzen der adsorbierten NGL aus dem beladenen absorbierenden Medium und Bilden von regeneriertem absorbierendem Medium,
(d) Transportieren des regenerierten absorbierenden Mediums zurück zu der Adsorptionseinheit (10) zur Wiederverwendung,
(e) Wiedergewinnen der freigesetzten NGL
und
(f) Abfackeln der methanreichen Erdgaszufuhr,
wobei das Adsorptionsmedium ein poröses vernetztes polymeres Adsorptionsmittel, ein teilweise pyrolysiertes makroporöses Polymer oder Mischungen davon ist.

2. Verfahren gemäß Anspruch 1, wobei der Erdgaszustrom von einer Ölbohrung, einer Gasbohrung oder einer Kondensatbohrung stammt.

3. Verfahren gemäß Anspruch 1, wobei das beladene Adsorptionsmedium mittels reduzierten Drucks über dem Medium, Erwärmen des Mediums oder einer Kombination von reduziertem Druck und Erwärmen regeneriert wird.

4. Verfahren gemäß Anspruch 1, wobei das beladene Adsorptionsmedium durch ein Mikrowellenerwärmungssystem (32) regeneriert wird.

5. Verfahren gemäß Anspruch 1, wobei der Regenerierschritt als ein diskontinuierlicher Prozess, ein halbkontinuierlicher Prozess oder als ein kontinuierlicher Prozess betrieben wird.

6. Verfahren gemäß Anspruch 1, wobei die wiedergewonnenen NGL per LKW, Schiene oder Schiff individuell oder als eine Mischung von Gasen (z. B. C₂, C₃, C₄, C₅ etc.) oder verflüssigt individuell oder als eine Mischung von Flüssigkeiten gelagert und/oder transportiert werden.

## Revendications

1. Une méthode pour séparer et récupérer certains ou tous les liquides de gaz naturel (LGN) : l'éthane, le propane, le butane, le pentane, ou des hydrocarbures plus lourds d'un flux d'alimentation en gaz naturel ce qui forme une arrivée de gaz naturel riche en méthane et brûler ensuite à la torche ladite arrivée de gaz naturel riche en méthane, où les LGN sont séparés du flux d'alimentation en gaz naturel par le biais d'une unité de séparation de LGN (90), l'unité de séparation de LGN (90) comprenant :
(i) une unité d'adsorption (10) comprenant un lit d'adsorption (2) comprenant un média adsorbant qui adsorbe des LGN afin de former un média adsorbant chargé
et
(ii) une unité de régénération (20) comprenant un moyen (32) afin de régénérer le média adsorbant chargé en causant la libération de LGN adsorbés du média adsorbant chargé et en formant le média adsorbant régénéré
où la méthode comprend les étapes consistant :
(a) à faire passer le flux d'alimentation en gaz naturel à travers l'unité d'adsorption (10) ce qui génère l'adsorbant chargé de LGN et une arrivée de gaz naturel riche en méthane,
(b) à transporter l'adsorbant chargé de LGN de l'unité d'adsorption (10) à l'unité de régénération (20),
(c) à régénérer l'adsorbant chargé de LGN en libérant les LGN adsorbés du média adsorbant chargé et en formant le média adsorbant régénéré,
(d) à transporter le média adsorbant régénéré en le faisant revenir à l'unité d'adsorption (10) pour être réutilisé,
(e) à récupérer les LGN libérés,
et
(f) à brûler à la torche l'arrivée de gaz naturel riche en méthane,
où le média d'adsorption est un adsorbant polymérique réticulé poreux, un polymère macroporeux partiellement pyrolysé, ou des mélanges de ceux-ci.

2. La méthode de la revendication 1 où le flux d'alimentation en gaz naturel provient d'un puits de pétrole, d'un puits de gaz, ou d'un puits de condensat.

3. La méthode de la revendication 1 où le média d'adsorption chargé est régénéré par le biais d'une pression réduite au-dessus du média, d'un chauffage du média, ou d'une combinaison d'une pression réduite et d'un chauffage.

4. La méthode de la revendication 1 où le média d'adsorption chargé est régénéré grâce à un système de chauffage par micro-ondes (32).

5. La méthode de la revendication 1 où l'étape de régénération est mise en oeuvre sous la forme d'un procédé discontinu, d'un procédé semi-continu, ou d'un procédé continu.

6. La méthode de la revendication 1 où les LGN récupérés sont stockés et/ou transportés par route, fer, ou mer individuellement ou sous la forme d'un mélange de gaz (par ex. sous la forme de C₂, C₃, C₄, C₅, etc.) ou liquéfiés individuellement ou sous la forme d'un mélange de liquides.
